Europäisches Patentamt

European Patent Office

Office européen des brevets

(11) Publication number: **0 205 072 B2**

(12) NEW EUROPEAN PATENT SPECIFICATION

(45) Date of publication of the new patent specification:
14.12.94

(51) Int. Cl.5: **A61M 16/00**, A61M 16/10, A61M 16/16

(21) Application number: **86107494.6**

(22) Date of filing: **03.06.86**

(54) Heat and moisture exchanging apparatus.

(30) Priority: **06.06.85 JP 123369/85**

(43) Date of publication of application:
**17.12.86 Bulletin 86/51**

(45) Publication of the grant of the patent:
**21.08.91 Bulletin 91/34**

(45) Mention of the opposition decision:
**14.12.94 Bulletin 94/50**

(84) Designated Contracting States:
**BE DE FR GB IT**

(56) References cited:
DE-B- 2 223 474      SE-A- 201 960
SE-A- 201 960        US-A- 3 231 409
US-A- 3 368 327      US-A- 3 982 095
US-A- 4 200 094      US-A- 4 381 267

(73) Proprietor: **TERUMO KABUSHIKI KAISHA trading as TERUMO CORPORATION**
**44-1, 2-chome, Hatagaya**
**Shibuya-Ku**
**Tokyo 151 (JP)**

(72) Inventor: **Nudeshima, Masahiro**
**379-1,Nakajima-cho**
**Fujinomiya-shi**
**Shizuoka (JP)**
Inventor: **Ohshima, Yasushi**
**Ken-ei-Fujimidaidanchi E202**
**4-1, Fujimidai**
**Fuji-shi**
**Shizuoka (JP)**

(74) Representative: **Henkel, Feiler, Hänzel & Partner**
**Möhlstrasse 37**
**D-81675 München (DE)**

EP 0 205 072 B2

**Description**

BACKGROUND OF THE INVENTION

This invention relates to an apparatus for warming and humidifying inspired gas to optimize its temperature and humidity in treating the patient using an anesthesia equipment or respirator.

Heat and moisture exchanging apparatus must meet the requirements of (1) a stable supply of sufficient heat and moisture to the inspired gas, (2) low and consistent pressure drop, (3) small dead volume, (4) small size and light weight, and the like.

Some typical examples of heat and moisture exchanging apparatus are disclosed in Japanese Utility Model Publication No. 52-3911, Japanese Patent Application Kokai Nos. 53-126796 and 55-73268 (or DE-A-2851564). Besides, there are known a number of such apparatus.

One prior art respiratory heat and moisture exchanger (see SE-A-201960) uses a corrugated paper web which is a laminate prepared by corrugating a thin sheet of paper and placing the corrugated sheet on another flat thin sheet of paper. A tape-like corrugated paper web having a certain width is spirally coiled into a roll, which is received in a cylindrical housing. Although this prior art apparatus is small sized and light weight, the initially coiled portion of the corrugated web, that is, the central portion of the roll tends to be too tight or too loose. It is thus difficult to establish a uniform fluid flow throughout the fluid passage area. This leads to the problems that the pressure drop is inconsistent, warming and humidifying functions are deteriorated, and a stable supply of sufficient heat and moisture is difficult.

SUMMARY OF THE INVENTION

Therefore, an object of the present invention is to provide a new and improved respiratory heat and moisture exchanger which can consistently supply sufficient heat and moisture to inspired gas in the process of anesthesia and artificial respiration, has a minimized dead volume, and develops an equal and low pressure drop over a transverse section to respiratory gas passage.

The present invention is directed to a respiratory gas warming and humidifying apparatus comprising a housing having a port to be connected to an endotracheal or tracheotomy tube on one side thereof and another port to be connected to a respiratory gas delivery tube on another side whereby respiratory gas is passed through the housing between the ports. According to a first aspect of the present invention, a heat and moisture accumulator comprising an alternate parallel stack of corrugated paper sheets and flat paper sheets is disposed in the housing whereby the gas is passed through the gaps between the corrugated sheets and the flat sheets.

According to a preferred embodiment of the present invention, at least the corrugated paper sheets, preferably both the corrugated and flat paper sheets of the heat and moisture accumulator have a hygroscopic material applied thereto.

In one preferred embodiment of the present invention, the corrugated paper sheet consists of a thin paper sheet of 0.01 to 0.30 mm thick. The sheet is corrugated to form ridges of 0.5 to 3.0 mm high at a pitch of 0.5 to 5.0 mm.

Preferably, the hygroscopic material is at least one member selected from the group consisting of glycerin, polyvinyl pyrrolidone, polyacrylic acid, polyacrylates, polyvinyl alcohol, glycols, potassium chloride, and lithium chloride.

The hygroscopic material is preferably contained in an amount of 5 to 50% by weight of the sheets.

BRIEF DESCRIPTION OF THE DRAWINGS

The above and other objects, features, and advantages of the invention will be seen by reference to the following description, taken in connection with the accompanying drawings, in which:

FIG. 1 is an elevational cross section of a respiratory heat and moisture exchanger according to one embodiment of the present invention;

FIG. 2 is a transverse cross section of the present apparatus of FIG. 1;

FIG. 3 is an enlarged perspective view of a corrugated paper sheet used in the present apparatus; and

FIG. 4 illustrates the actual use of a respiratory heat and moisture exchanger.

## DETAILED DESCRIPTION OF THE PREFERRED EMBODIMENTS

In the following description, the respiratory heat and moisture exchanger is hereinafter referred to as a heater/humidifier for brevity's sake.

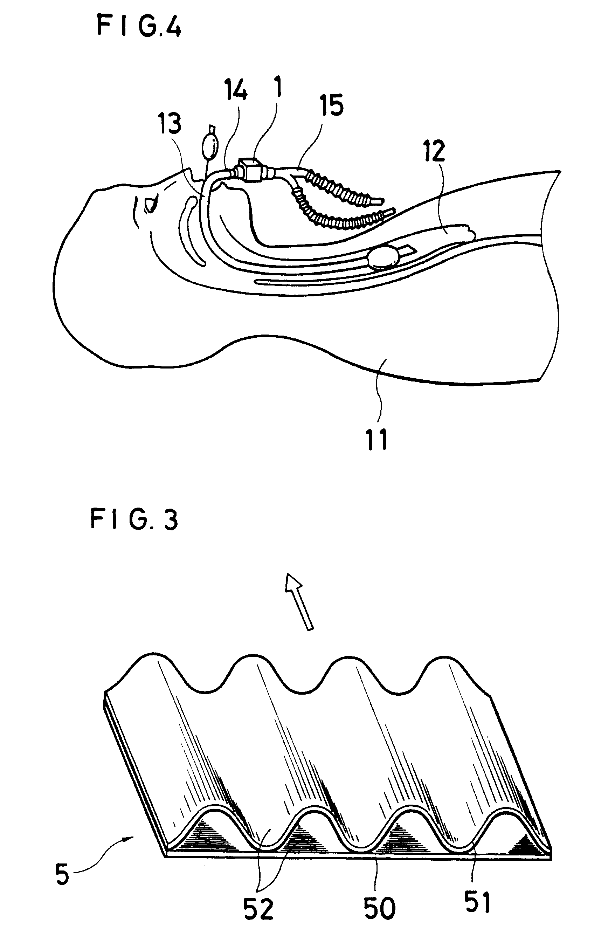

FIG. 4 illustrates how to use respiratory heat and moisture exchanger in the actual treatment of the patient. An endotracheal tube 13 is inserted into the trachea 12 of the patient 11. Respiratory heat and moisture exchanger designated at 1 is interposed between a connector 14 mounted on the proximal end of the endotracheal tube 13 and a hose 15 connected to a respiratory gas source (not shown) such as an anesthesia equipment or respirator.

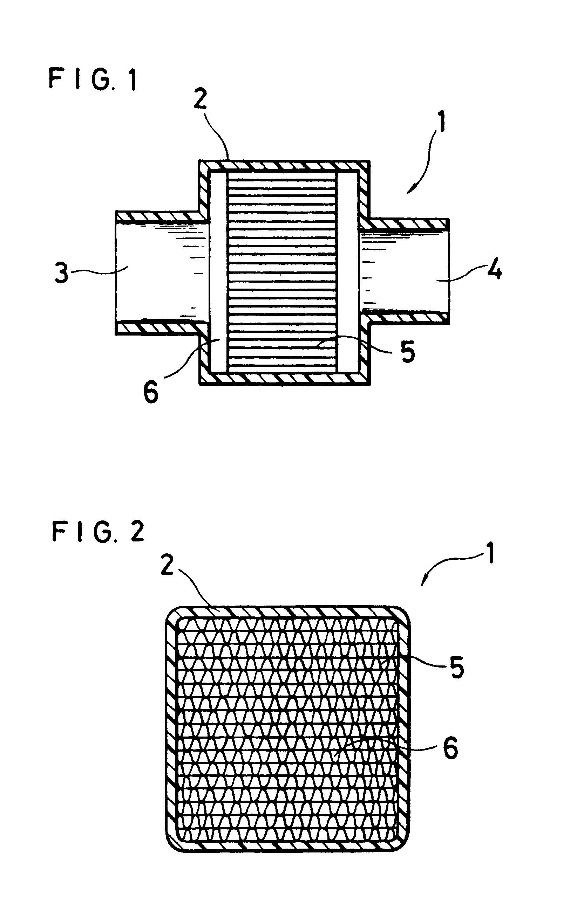

FIGS. 1 and 2 are elevational and transverse cross sections of the heater/humidifier 1, respectively. The heater/humidifier 1 includes a housing 2 of a rectangular cross section which receives a heat and moisture accumulator 6 in its cavity. The heat and moisture accumulator 6 is a stack of parallel laminated corrugated paper boards 5 as shown in FIG. 2. Each corrugated paper board 5 consists of a flat paper sheet 50 and a corrugated paper sheet 51 as shown in FIG. 3. Therefore, the corrugated paper sheets and flat paper sheets are alternately placed one on another in parallel in the housing cavity as best shown in FIG. 2.

The housing 2 on one side has a port 3 to be connected to an endotracheal or tracheotomy tube, for example, the endotracheal tube 13. The housing on the axially opposed side has another port 4 to be connected to a respiratory gas delivery tube, for example, the hose 15 in the illustrated embodiment.

Expired gas incoming from the port 3 passes through the heat and moisture accumulator 6 and exits from the other port 4. Reversely, inspired gas incoming from the other port 4 passes through the heat and moisture accumulator 6 and exits from the port 3. During passage through the accumulator, the expired gas gives up heat and moisture to the accumulator 6 and the inspired gas receives heat and moisture from the accumulator 6, providing a respiration process close to the spontaneous respiration.

The heat and moisture accumulator 6 used herein is of the structure wherein corrugated paper boards 5 each consisting of a flat paper sheet 50 and a corrugated paper sheet 51 are stacked one on top of another to form a parallel laminate as shown in FIGS. 2 and 3. The corrugated board 5 has a plurality of transverse ridges and flutes which define gaps 52. Each gap 52 constitutes a flowpath for respiratory gas. Preferably the flat sheet 50 is bonded to the corrugated sheet 51 at the apex of its flutes because the resulting corrugated board is resistant to deformation.

The stack or laminate of the corrugated boards 5 is preferably placed in the housing 2 such that the gaps 52 or flowpaths extend parallel to an axial direction of the housing (a direction connecting the ports), that is, the flow direction of respiratory gas shown by the arrow in FIG. 3. The gaps 52 may be slant as long as the pressure drop is little increased. The gaps 52 need not be straight as long as they extend from one edge to the opposite edge of the board. For example, the gaps may be curved or serpentine.

The heat and moisture accumulator 6 assembled by stacking a plurality of corrugated boards 5 in parallel has a honeycomb-like structure where efficient exchange of heat and moisture takes place and a substantially equal pressure drop develops over the entire fluid passage area.

It was difficult for the conventional heat and moisture accumulator to develop a uniform pressure drop because it is a spiral roll of a tape-like corrugated web whose central portion tends to be too tight or too loose. Since the heat and moisture accumulator 6 used in the present invention is a parallel stack of the corrugated boards 5 wherein the corrugations are regularly disposed and the gaps 52 having substantially the same volume are defined, the pressure drop across the accumulator is uniform over the entire fluid passage area.

It is desired that the housing 2 is fully charged with the corrugated board 5 stack without leaving an empty space so that all incoming fluid passes through the accumulator 6.

The dimensions of the corrugated board 5, particularly corrugated sheet 51 may be adequately determined so as to meet a particular demand. Preferred is a corrugated sheet fabricated by corrugating a thin paper sheet of 0.01 to 0.30 mm thick to form ridges of 0.5 to 3.0 mm high at a pitch of 0.5 to 5.0 mm because of improved warming and humidifying performance.

Thinner paper sheets having a thickness of less than 0.01 mm offer a poor warming and humidifying ability. Thicker sheets having a thickness of more than 0.30 mm develop an increased pressure loss and a low warming and humidifying ability. The height of the ridges or flutes ranges from 0.5 mm to 3.0 mm because lower ridges define narrow gaps across which an increased pressure drop develops. Ridges higher than 3.0 mm do not provide a satisfactory warming and humidifying ability. Similarly, corrugation pitches of less than 0.5 mm results in an increased pressure drop whereas pitches of more than 5.0 mm do not provide a satisfactory warming and humidifying ability.

The materials of which the corrugated boards 5, more particularly, sheets 50 and 51 are made include plant fibers, viscose rayons, celluloses, and acetates, and a variety of synthetic fibers such as fibrous polyamides, polyesters, polyurethanes, polyacrylonitriles, and polyvinyl alcohols. The materials are not limited to these examples as long as they can be made into corrugatable paper.

The corrugated boards 5, more particularly sheets 50 and 51 have sufficient heat accumulating and moisture absorbing or hygroscopic capacities in themselves. In order to enhance the hygroscopicity, the corrugated boards 5 contain a hygroscopic material. More particularly, the hygroscopic material should preferably be contained in the corrugated sheets and most preferably in both the corrugated and flat sheets 51 and 50.

The hygroscopic materials used herein must have the following physical and chemical properties. The hygroscopic materials are less toxic materials including liquids miscible with water or alcohols in any proportion and nonvolatile at room temperature and solids soluble in water or alcohols. They must have the hygroscopic ability of providing a moisture content of at least 5% under normal conditions (temperature 20° C, humidity 65%) and readily releasing the once absorbed moisture.

Some preferred, non-limiting examples of the hygroscopic materials include glycerin, polyvinyl pyrrolidone, polyacrylic acid, polyacrylate salts, polyvinyl alcohol, glycols, potassium chloride, and lithium chloride, with the glycerin being most preferred. The hygroscopic materials may be used alone or in combination of two or more.

The hygroscopic materials may be applied to the paper sheets by any desired conventional techniques, for example, by impregnating the sheets with the materials or coating the surface of the sheets with the materials. To meet the required performance of heat and moisture exchanger, the hygroscopic mateirals are preferably contained in amounts of 5 to 50% by weight of the sheets. No substantial improvement in humidifying capacity is available with contents of less than 5% by weight. Contents of more than 50% by weight increase the pressure drop. In addition to the content of the hygroscopic material falling within the above-defined range, it should, of course, be uniformly distributed throughout the resultant accumulator 6.

The operation of respiratory heat and moisture exchanger of the present invention will be described.

In the anesthesia or respiration process to the patient, respiratory heat and moisture exchanger 1 is interposed between the endotracheal or tracheotomy tube 13 inserted into the trachea 12 of the patient 11 and the hose 15 connected to a respiratory gas source (not shown) such as an anesthesia equipment or respirator in order to provide respiration conditions approaching the spontaneous respiration. The endotracheal or tracheotomy tube 13 is connected to the port 3 in the housing 2 of respiratory heat and moisture exchanger 1 through the connector 14, and the hose 15 connected to the other port 4. When respiration is conducted on the patient with this setting, expired and inspired gases are alternately passed through the heater/humidifier 1.

At the time of expiration, expired gas flows into the housing 2 through the port 3 where it passes the gaps 52 in and between the corrugated boards 5 of the stack accumulator. The heat of the expired gas is transferred to the corrugated boards 5 whereby the expired gas itself cools and oversaturated moisture condenses into water droplets which deposit on the corrugated boards 5. As a result, the corrugated boards 5 accumulate heat and moisture.

The corrugated boards 5 containing an optimum amount of the hygroscopic material exhibit an enhanced moisture absorbing capacity because the hygroscopic material can additionally accumulate a more amount of moisture.

At the time of inhalation, inspired gas flows into the housing 2 through the other port 4 where it passes through the gaps 52 in and between the corrugated boards 5 of the stack or accumulator 6. The heat and moisture accumulated in the corrugated boards 5 are released and transferred to the inspired gas whereby the inspired gas is warmed and humidified to conditions close to the spontaneous respiration.

Upon passage through the accumulator 6, the expired and inspired gases undergo a uniform pressure drop over the entire fluid passage area because the corrugated boards 5 are placed in a regular and parallel stack form.

Example 1

To a respiratory heat and moisture exchanger having the following specifications were connected an endotracheal tube and a hose.

(1) Housing

| Volume: | 9 ml |
| Inside dimensions: | fluid passage area 25 mm x 25 mm |

4

(2) Corrugated boards

| Material: | cellulose |
|---|---|
| Sheet thickness: | 0.15 mm |
| Ridge height: | 1 mm |
| Ridge pitch: | 3 mm |
| Total surface area: | 300 cm$^2$ |
| Arrangement: | parallel stack in the housing |
| Alignment: | hollow gaps extending in parallel with respiratory gas flow direction |

(3) Hygroscopic material
Not used

Example 2

Respiratory heat and moisture exchanger was fabricated using the same specifications as in Example 1 except that glycerin as the hygroscopic material was uniformly contained in the stack of corrugated boards in an amount of 20% by weight.

Example 3

This example is a comparative example. Respiratory heat and moisture exchanger was fabricated using the same specifications as in Example 1 except that a length of similar corrugated web was spirally wound into a roll which was placed in a cylindrical housing having an inner diameter of 28 mm.

Artificial respiration was carried out through the units of Examples 1 to 3 to determine the amounts of heat and moisture imparted to inspiring gas and the pressure drop across the units. The respiration process was effected using an expiring gas having a temperature of 32° C and a relative humidity of 100% and an inspiring gas having a temperature of 20° C and a relative humidity of 25 to 40%. The temperature and absolute humidity of the inspiring gas after passage from the unit were measured.

The displacement of one cycle was about 500 ml and 10 respiration cycles per minute were carried out. The measurements are averaged. The results are shown in Table 1.

Table 1

| Example | Temperature (°C) | Absolute humidity (mg/l) | Pressure drop (cm $H_2O$/l/sec.) |
|---|---|---|---|
| 1 | 26.2 | 23 | 1.0 |
| 2 | 28.2 | 28 | 1.1 |
| 3 | 26.2 | 23 | 1.6 |

As seen from the data in Table 1, in the present examples, particularly Example 2, inspiring gas has temperature and humidity close to those at the top of the trachea during spontaneous respiration so that a minimized burden is laid on the living body, proving the benefits of the present invetion.

The respiratory heat and moisture exchanger of the present invention have the following benefits.

(1) Since expired and inspired gases flow through a regular and parallel stack of corrugated boards occupying substantially the entire volume of a housing, an increased surface area for heat and moisture exchange is available in a certain volume and an enhanced warming and humidifying capacity is obtained. Respiratory conditions closely resembling the spontaneous respiration are maintained in a stable manner.

This advantage becomes more outstanding particularly when corrugated paper sheets of the corrugated boards are prepared by corrugating a thin paper sheet of 0.01 to 0.30 mm thick to form ridges of 0.5 to 3.0 mm high at a pitch of 0.5 to 5.0 mm.

(2) The heat and moisture accumulator is of a regular honeycomb-like structure across which only a low and uniform pressure drop develops over the entire fluid passage area.

(3) A more enhanced warming and humidifying capacity is obtained when the hygroscopic material is applied to or contained in the corrugated boards. Since the hygroscopic material possesses a certain amount of moisture prior to use, optimum humidifying is done from the start of operation because of the release of the originally possessed moisture.

(4) When the hygroscopic materials selected from glycerin, polyvinyl pyrrolidone, polyacrylic acid, polyacrylates, polyvinyl alcohol, glycols, potassium chloride, and lithium chloride are present in amounts of 5 to 50% by weight of the corrugated boards, the resultant accumulator has remarkably increased heat and moisture accumulation efficiency and capacity per unit volume. Products exhibiting a stabilized warming and humidifying performance are consistently obtained.

(5) Since the respiratory heat and moisture exchanger has a simple construction that a plurality of corrugated boards are placed one on another in a housing, it can be manufactured at low cost and requires little maintenance operation or cost, contributing to a reduction of clinical treatment cost.

(6) The dead volume where carbon dioxide gas would accumulate is minimized and the gas flow experiences little compression, providing an accurate control of displacement.

## Claims

1. A heat and moisture exchanging apparatus comprising a housing having a port to be connected to an endotracheal or tracheotomy tube on one side thereof and another port to be connected to a gas delivery means on another side whereby the gas is passed through the housing between the ports, wherein

   a heat and moisture accumulator comprising an alternate parallel stack of corrugated sheets and flat sheets is disposed in said housing such that the gas passes through the gaps between the corrugated sheets and the flat sheets.

2. A heat and moisture exchanging apparatus according to claim 1 wherein each said corrugated sheet consists of a thin paper sheet of 0.01 to 0.30 mm thick, the sheet being corrugated to form ridges of 0.5 to 3.0 mm high at a pitch of 0.5 to 5.0 mm.

3. A heat and moisture exchanging apparatus according to claim 1 wherein at least the corrugated sheets of said heat and moisture accumulator have a hygroscopic material applied thereto.

4. A heat and moisture exchanging apparatus according to claim 3 wherein said hygroscopic material is at least one member selected from the group consisting of glycerol, polyvinyl pyrrolidone, polyacrylic acid, polyacrylates, polyvinyl alcohol, glycols, calcium chloride, and lithium chloride.

5. A heat and moisture exchanging apparatus according to claim 3 or 4 wherein said hygroscopic material is contained in an amount of 5 to 50% by weight of the sheets.

6. A heat and moisture exchanging apparatus according to claim 1 wherein both the corrugated sheets and the flat sheets of said heat and moisture accumulator have a hygroscopic material applied thereto.

7. A heat and moisture exchanging apparatus according to claim 6 wherein said hygroscopic material is at least one member selected from the group consisting of glycerol, polyvinyl pyrrolidone, polyacrylic acid, polyacrylates, polyvinyl alcohol, glycols, calcium chloride, and lithium chloride.

8. A heat and moisture exchanging apparatus according to claim 6 or 7 wherein said hygroscopic material is contained in an amount of 5 to 50% by weight of the sheets.

## Patentansprüche

1. Wärme- und Feuchtigkeitstauscher, umfassend ein Gehäuse mit einer an ein Endotracheal- oder Tracheotomierohr anzuschließenden Öffnung auf einer Seite und einer an eine Gasabgabeeinrichtung anzuschließenden weiteren Öffnung auf der anderen Seite, wobei das Gas zwischen den Öffnungen durch das Gehäuse geleitet wird, wobei

   in dem Gehäuse ein Wärme- und Feuchtigkeitssammler mit einem abwechselnden parallelen Stapel gewellter Lagen und flacher Lagen derart angeordnet ist, daß das Gas durch die Spalte zwischen den gewellten Lagen und den flachen Lagen hindurchtritt.

2. Wärme- und Feuchtigkeitstauscher nach Anspruch 1, wobei jede dieser gewellten Lagen aus einem dünnen Blatt Papier einer Stärke von 0,01 - 0,30 mm besteht und das Blatt derart gewellt ist, daß sich Kanten einer Höhe von 0,5 - 3,0 mm mit einem Teilungsabstand von 0,5 - 5,0 mm bilden.

3. Wärme- und Feuchtigkeitstauscher nach Anspruch 1, wobei mindestens die gewellten Lagen des Wärme- und Feuchtigkeitssammlers ein hygroskopisches Material appliziert enthalten.

4. Wärme- und Feuchtigkeitstauscher nach Anspruch 3, wobei das hygroskopische Material aus mindestens einem Bestandteil aus der Gruppe Glycerin, Polyvinylpyrrolidon, Polyacrylsäure, Polyacrylate, Polyvinylalkohol, Glykole, Calciumchlorid und Lithiumchlorid besteht.

5. Wärme- und Feuchtigkeitstauscher nach Anspruch 3 oder 4, wobei das hygroskopische Material in einer Menge von 5 - 50 Gew.-% der Lagen vorhanden ist.

6. Wärme- und Feuchtigkeitstauscher nach Anspruch 1, wobei sowohl die gewellten Lagen als auch die flachen Lagen des Wärme- und Feuchtigkeitssammlers ein hygroskopisches Material appliziert enthalten.

7. Wärme- und Feuchtigkeitstauscher nach Anspruch 6, wobei das hygroskopische Material aus mindestens einem Bestandteil aus der Gruppe Glycerin, Polyvinylpyrrolidon, Polyacrylsäure, Polyacrylate, Polyvinylalkohol, Glykole, Calciumchlorid und Lithiumchlorid besteht.

8. Wärme- und Feuchtigkeitstauscher nach Anspruch 6 oder 7, wobei das hygroskopische Material in einer Menge von 5 - 50 Gew.-% der Lagen vorhanden ist.

**Revendications**

1. Echangeur de chaleur et d'humidité comprenant un boîtier muni d'un côté d'un orifice à relier à un tube endotrachéal ou un tube de trachéotomie et de l'autre côté d'un autre orifice à relier à un dispositif d'apport de gaz, le gaz traversant le boîtier entre les orifices, dans lequel un accumulateur de chaleur et d'humidité constitué par un empilement parallèle et alterné de feuilles ondulées et de feuilles planes est disposé dans ledit boitier de façon telle que le gaz passe par les intervalles entre les feuilles ondulées et les feuilles planes.

2. Echangeur de chaleur et d'humidité selon la revendication 1, dans lequel chacune desdites feuilles ondulées est constituée par une mince feuille de papier de 0,01 à 0,30 mm d'épaisseur, la feuille étant ondulée de façon à former des arêtes de 0,5 à 3,0 mm de haut, espacées de 0,5 à 5,0 mm.

3. Echangeur de chaleur et d'humidité selon la revendication 1, dans lequel un matériau hygroscopique est appliqué au moins sur les feuilles ondulées dudit accumulateur de chaleur et d'humidité.

4. Echangeur de chaleur et d'humidité selon la revendication 3, dans lequel ledit matériau hygroscopique est au moins une substance sélectionnée dans le groupe comprenant le glycérol, la polyvinylpyrrolidone, l'acide polyacrylique, les polyacrylates, l'alcool polyvinylique, les glycols, le chlorure de calcium et le chlorure de lithium.

5. Echangeur de chaleur et d'humidité selon la revendication 3 ou 4, dans lequel ledit matériau hygroscopique est présent en une quantité représentant 5 à 50 % en poids des feuilles.

6. Echangeur de chaleur et d'humidité selon la revendication 1, dans lequel un matériau hygroscopique est appliqué sur les feuilles ondulées et les feuilles planes dudit échangeur de chaleur et d'humidité.

7. Echangeur de chaleur et d'humidité selon la revendication 6, dans lequel ledit matériau hygroscopique est au moins une substance sélectionnée dans le groupe comprenant le glycérol, la polyvinylpyrrolidone, l'acide polyacrylique, les polyacrylates, l'alcool polyvinylique, les glycols, le chlorure de calcium et le chlorure de lithium.

8. Echangeur de chaleur et d'humidité selon la revendication 6 ou 7, dans lequel ledit matériau hygroscopique est présent en une quantité représentant 5 à 50 % en poids des feuilles.

F I G. 1

F I G. 2

FIG.4

FIG. 3